# EUROPEAN PATENT APPLICATION

(11) **EP 1 164 161 A1**
(43) Date of publication of application: **19.12.2001**
(21) Application number: 00112769.5
(22) Date of filing: 16.06.2000
(51) Int. Cl.: C08K 5/134, A61L 15/22

(54) **Thermoplastic hydrophilic polymeric compositions with high water solubility component**

(71) Applicant: The Procter & Gamble Company, Cincinnati, Ohio 45202 (US)
(72) Inventor: Corzani, Italo, 66100 Chieti (IT); Russo, Elisabetta, 00142 Rom (IT); Sirianni, Giuseppe, 00165 Rom (IT)
(74) Representative: Veronese, Pancrazio

(57) **Abstract**

The present invention relates to thermoplastic hydrophilic polymeric compositions for making a liquid impermeable moisture vapour permeable structure, for example by forming the composition into a layer or film. The thermoplastic compositions comprise preferred thermoplastic polymers and suitable compatible plasticisers which allow a controlled extraction/release by water or aqueous fluids. The layers made from the thermoplastic hydrophilic polymeric compositions of the present invention can find a variety of applications wherein liquid imperviousness and moisture vapour permeability are desirable.

## Description

### Field of the Invention

The present invention relates to thermoplastic hydrophilic polymeric compositions for making a moisture vapour permeable, liquid impermeable structure, for example by forming the composition into a moisture vapour permeable, liquid impermeable layer such as a film. The thermoplastic compositions of the present invention and the moisture vapour permeable, liquid impervious structures formed therefrom find utility in a number of applications wherein liquid imperviousness and moisture vapour permeability are desirable.

### Background of the Invention

Thermoplastic films which provide a liquid barrier in addition to providing moisture vapour permeability are known in the art. Particularly preferred are hydrophilic continuous films that do not allow the flow of moisture vapour through open pores or apertures in the material, but do transfer substantial amounts of moisture vapour through the film by absorbing water on one side of the film where the moisture vapour concentration is higher, and desorbing or evaporating it on the opposite side of the film where the moisture vapour concentration is lower. Such films are typically formed from a thermoplastic hydrophilic polymeric composition comprising a thermoplastic hydrophilic polymer, or a blend of thermoplastic hydrophilic polymers. Thermoplastic hydrophilic polymeric compositions having the above described characteristics are also known in the art as "monolithic compositions", and the moisture vapour permeable, liquid impermeable layers or films made therefrom are known as "monolithic layers" or "monolithic films".

For example WO 95/16746 discloses films prepared from mixtures of a) block copolyether ester, block copolyether amides (e.g. Pebax™) and or polyurethane and b) thermoplastic polymer which is incompatible with a, and c) a compatibiliser. The films are liquid impermeable and have moisture vapour permeability of about 700 g/m²·day. Also, US 5,447,783 discloses a vapour permeable water resistant multi component film structure having at least three layers. The outer layers are hydrophobic copolyetherester elastomers having a thickness of 1.3-7.6 micrometers and a WVTR of 400-2500 g/m²·24h and the inner layer is a hydrophilic copolyetherester elastomer having a thickness of 7.6 - 152 micrometers and a WVTR of at least 3500 g/m²·24h.

US 5,445,875 discloses a waterproof, bloodproof and virusproof breathable laminate. The laminate comprises a woven/nonwoven fabric and an extruded film such as Hytrel™ having a thickness of about 1mil (25.4 micrometers).

US 5,532,053 discloses a high moisture transmission medical film which can be laminated onto a nonwoven material. The laminate film comprises a first layer of polyetherester copolymer and second and third layers selected from a specified group of polymers. The film has a MVTR of greater than 750 g/m²·24h (ASTM F1249) and a thickness of less than 1 mil (25.4 micrometer) preferably 0.6 mil to 0.75 mil (15-19 micrometers).

US 4,938,752 discloses absorbent articles comprising films of copolyether esters which have reduced water permeability, a water vapour permeability of 500 g/m²·24h (as measured in a specified described test) and a thickness of 5-35 micrometers. There is no disclosure of a supportive substrate.

US 4,493,870 discloses a flexible layered waterproof product comprising a textile material covered with a film of a copolyetherester having an MVTR of at least 1000 g/m²·24h (ASTM E96- 66) having a thickness of 5 to 35 micrometers.

GB 2024100 discloses a flexible layered water resistant article comprising a microporous hydrophobic outer layer which is moisture vapour permeable but resist liquids and a hydrophilic inner layer of polyetherpolyurethane having a MVTR of above 1000 g/m²·24h.

In our patent applications WO 99/64077 entitled "Low viscosity thermoplastic compositions for moisture vapour permeable structures and the utilisation thereof in absorbent articles", and WO 99/64505 entitled "Low viscosity thermoplastic compositions for structures with enhanced moisture vapour permeability and the utilisation thereof in absorbent articles", thermoplastic compositions comprising a thermoplastic hydrophilic polymer, or a blend of thermoplastic hydrophilic polymers, are disclosed for making hydrophilic continuous moisture vapour permeable, liquid impermeable layers having preferred characteristics of moisture vapour permeability and liquid imperviousness. The disclosed preferred thermoplastic compositions are also readily processable so as to provide a coating having the desired thickness onto a substrate, so avoiding the need of complex traditional extrusion apparatuses. This is achieved by modifying the viscosity of the thermoplastic hydrophilic polymers by means of the inclusion in the composition of a suitable plasticiser or blend of plasticisers that lowers such viscosity. This allows to utilise with these preferred compositions typical process conditions known in the art for the direct coating of low viscosity hot melt compositions onto a substrate in order to form a moisture vapour permeable, liquid impervious film or layer.

Particularly preferred hydrophilic plasticisers are described in WO 99/64505, which, in addition to adjusting the viscosity of the compositions, also provide the thermoplastic hydrophilic polymeric compositions with a further benefit in terms of moisture vapour permeability.

While the thermoplastic polymeric hydrophilic compositions ("monolithic compositions") described in WO 99/64077 and WO 99/64505 are suitable for making moisture vapour permeable, liquid impermeable films or layers having a high moisture vapour permeability combined with liquid imperviousness, wherein at the same time said compositions are also readily processable, they can still be improved in terms of their processability and of their possible interactions with the environment during the use of said films or layers.

Thermoplastic hydrophilic polymeric compositions according to the above mentioned patent applications comprise plasticisers which are highly effective in adjusting the viscosity of the compositions at the process conditions, preferably also providing a benefit in terms of moisture vapour permeability of the films or layers produced therefrom.

While it is generally required that plasticisers form stable blends with other components of the thermoplastic hydrophilic polymeric compositions, particularly with the thermoplastic hydrophilic polymer or polymers, there are cases in which a controlled extraction/release of the plasticiser or plasticisers from the composition is desired.

For example, it can be preferred to have a higher percentage of plasticiser or plasticisers in the thermoplastic hydrophilic polymeric composition in order to enhance processability of said composition, e.g. when forming it into films or layers. After formation of the thermoplastic hydrophilic polymeric composition into a structure, e.g. into the above mentioned film or layer, it can be desired to reduce the plasticiser or plasticisers content in the composition, for example in order to modify the mechanical characteristics of the composition itself, and in turn of the structure made of the composition.

Alternatively, it can be desired to have a controlled extraction/release of the plasticiser or plasticisers during the use of the structure made of the thermoplastic hydrophilic polymeric composition, in order to take advantage for example of some specific activity of the plasticiser or plasticisers towards other surfaces or objects intended to come in contact with the structure during its use, for example humans, animals, food or other goods.

It has been discovered that some plasticisers can have a rather high sensitivity to water and aqueous fluids, and can be extracted form the composition upon contact with water or aqueous fluids.

It is therefore an object of the present invention to provide thermoplastic hydrophilic polymeric compositions for moisture vapour permeable, liquid impermeable structures, which have the desired moisture vapour permeability and liquid imperviousness, and wherein controlled extraction/release of plasticiser or plasticisers upon direct contact of the structure with water or aqueous fluids is made possible.

It has surprisingly been discovered that this can be achieved by selecting, for said thermoplastic hydrophilic polymeric compositions, a suitable compatible plasticiser or a blend of plasticisers having a preferred solubility in water.

### Summary of the Invention

The present invention relates to a thermoplastic hydrophilic polymeric composition for making a moisture vapour permeable, liquid impervious structure. The composition comprises:
- a thermoplastic hydrophilic polymer or mixture of polymers selected from the group consisting of polyurethanes, poly-ether-amides block copolymers, polyethylene-acrylic acid copolymers, polyethylene oxide and its copolymers, ethylene acrylic esters copolymers, poly lactide and copolymers, polyamides, polyester block copolymers, sulfonated polyesters, poly-ether-ester block copolymers, poly-ether-ester-amide block copolymers, polyacrylates, polyacrylic acids and derivatives, ionomers, polyethylene-vinyl acetate with a vinyl acetate content of more than 28% by weight, polyvinyl alcohol and its copolymers, polyvinyl ethers and their copolymers, poly-2-ethyl-oxazoline and derivatives, polyvinyl pyrrolidone and its copolymers, thermoplastic cellulose derivatives, or mixtures thereof;
- a suitable compatible plasticiser or blend of suitable compatible plasticisers, wherein the suitable compatible plasticiser has a solubility in water at 25°C of more than 6 g/100 ml H₂O, preferably of more than 7 g/100 ml H₂O, more preferably of more than 9 g/100 ml H₂O, or alternatively the blend of suitable compatible plasticisers contains more than 50% by weight of said blend of at least a plasticiser having a solubility in water at 25°C of more than 6 g/100 ml H₂O, preferably of more than 7 g/100 ml H₂O, more preferably of more than 9 g/100 ml H₂O.

### Detailed Description of the Invention

According to the present invention, the thermoplastic polymeric hydrophilic composition for making a moisture vapour permeable, liquid impervious structure at least comprises a thermoplastic hydrophilic polymer or a mixture of thermoplastic hydrophilic polymers, and a suitable compatible plasticiser, or a blend of suitable compatible plasticisers.

The terms "breathable" and "breathability" are intended herein to correspond to "moisture vapour permeable" or "water vapour permeable", and "moisture vapour permeability" or "water vapour permeability" respectively, with reference to "monolithic compositions" and "monolithic layers or films" as defined in the Background of the Invention. "Moisture vapour" and "water vapour" are also considered to be equivalent.

Suitable thermoplastic hydrophilic polymers comprised in the composition according to the present invention include polyurethanes, poly-ether-amides block copolymers, polyethylene-acrylic acid copolymers, polyethylene oxide and its copolymers, poly lactide and copolymers, polyamides, polyester block copolymers, sulfonated polyesters, poly-ether-ester block copolymers, poly-ether-ester-amide block copolymers, polyacrylates, polyacrylic acids and derivatives, ionomers, polyethylene-vinyl acetate with a vinyl acetate content of more than 28 weight %, polyvinyl alcohol and its copolymers, polyvinyl ethers and their copolymers, poly-2-ethyl-oxazoline and derivatives, polyvinyl pyrrolidone and its copolymers, thermoplastic cellulose derivatives, and mixtures thereof.

Particularly preferred thermoplastic hydrophilic polymers are thermoplastic poly-ether-amide block copolymers (e.g. Pebax™), thermoplastic poly-ether-ester-amide block copolymers, thermoplastic polyester block copolymers (e.g. Hytrel™), thermoplastic polyurethanes (e.g. Estane™), or mixtures thereof.

The thermoplastic hydrophilic polymeric compositions according to the present invention comprise a thermoplastic hydrophilic polymer or a mixture of thermoplastic hydrophilic polymers as mentioned above, and a suitable compatible plasticiser, or a blend of suitable compatible plasticisers.

According to the present invention, the suitable compatible plasticiser, or alternatively the blend of suitable compatible plasticisers, comprised in the thermoplastic hydrophilic polymeric composition must be selected such that they have a preferred solubility in water, in order to allow a controlled extraction/release of said plasticiser or plasticisers upon direct contact of water or aqueous fluids with structures, e.g. films or layers, made of said thermoplastic hydrophilic polymeric composition, after manufacture or during the use thereof.

When a single plasticiser is comprised in a thermoplastic hydrophilic polymeric composition according to the present invention, said plasticiser must be selected such that it has a solubility in water at 25°C of more than 6 g/100 ml H₂O, preferably of more than 7 g/100 ml H₂O, more preferably of more than 9 g/100 ml H₂O. Alternatively, when a blend of plasticisers is present in the thermoplastic polymeric hydrophilic composition of the present invention, said blend must contain more than 50% by weight of said blend of at least a plasticiser having a solubility in water at 25°C of more than 6 g/100 ml H₂O, preferably of more than 7 g/100 ml H₂O, more preferably of more than 9 g/100 ml H₂O.

Solubility of a substance in water at a given temperature is a parameter well known in chemistry and, according for example to the definition given on page F-101 of the CRC Handbook of Chemistry and Physics, CRC Press, Inc., 65^{th} Edition 1984-1985, corresponds to the mass of said substance contained in a solution which is in equilibrium with an excess of the substance; under which conditions the solution is said to be saturated. It is herein expressed as grams of the substance in 100 ml of water (g/100 ml H₂O)

The suitable compatible plasticiser, or the blend of suitable compatible plasticisers, in the thermoplastic hydrophilic polymeric compositions according to the present invention can be advantageously selected according to the above criteria among suitable compatible plasticisers which are known in the art.

In a preferred embodiment of the present invention the suitable compatible plasticiser, or the blend of suitable compatible plasticisers can be selected among the preferred plasticisers disclosed in our patent applications WO 99/64077 and WO 99/64505 and also mentioned above, which plasticisers are generally intended to adjust the viscosity of the thermoplastic hydrophilic polymeric compositions at the process conditions, and preferably to further provide a benefit in terms of moisture vapour permeability of the films or layers produced therefrom.

The thermoplastic hydrophilic polymers or mixture of thermoplastic hydrophilic polymers as mentioned above, comprised in the thermoplastic hydrophilic polymeric composition of the present invention, can be typically highly viscous in the melted state at the process conditions that are typical of the known processes of film or layer formation, e.g. an extrusion process involving a high power screw extruder. For example they may have a viscosity higher than 5000 poise at a temperature of 20°C above the DSC (Differential Scanning Calorimetry) melting point, which is the temperature identified as that corresponding to the DSC peak, or corresponding to the highest DSC peak in case of a mixture of polymers showing more than one peak, and at a frequency of 1 rad/sec.

The thermoplastic hydrophilic polymeric compositions of the present invention, comprising the thermoplastic hydrophilic polymer(s), can therefore still be highly viscous in the melted state at the process conditions.

According to a preferred embodiment of the present invention, and as disclosed in our patent applications WO 99/64077 or WO 99/64505, the viscosity of the thermoplastic polymeric hydrophilic compositions of the present invention can be typically adjusted by including in the thermoplastic hydrophilic polymeric composition a suitable plasticiser, or blend of plasticisers, that is also compatible with the thermoplastic hydrophilic polymer or polymers and that lowers the viscosity of the thermoplastic hydrophilic polymeric composition in the melted state at the process conditions.

Viscosity of the thermoplastic hydrophilic polymeric compositions of the present invention can therefore be typically adjusted to the desired level, depending on how the composition is to be processed. For example film extrusion techniques can be suitably used with compositions having higher viscosity at the process conditions, as it is known in the art. Alternatively, suitable hot melt coating processes can be preferred to process the compositions, as explained in the above mentioned patent applications WO 99/64077 and WO 99/64505. This implies that the viscosity in the thermoplastic hydrophilic polymeric composition at the process conditions has to be adjusted at a suitable lower level.

In such a case, the thermoplastic polymeric hydrophilic compositions of this alternative embodiment of the present invention comprise a suitable plasticiser or blend of plasticisers such that they preferably have the following complex viscosities (η∗):

50 poise < η∗ < 4000 poise, preferably 100 poise < η∗ < 2000 poise, more preferably 100 poise < η∗ < 1000 poise, at a frequency of 1 rad/s at a temperature of 210°C or less and η∗ < 2000 pose, preferably η∗ < 1000 poise, more preferably η∗ < 500 poise, at a frequency of 1000 rad/s at a process temperature (T) of 210°C or less, wherein η∗ represents the complex viscosity of the thermoplastic polymeric hydrophilic composition. Preferably the temperature T is 200°C or less and more preferably 180°C or less and most preferably from 200°C to 50°C.

According to this preferred embodiment of the present invention the thermoplastic hydrophilic polymeric compositions having the complex viscosity described above allow for a film or layer to be coated onto a substrate using typical coating conditions and apparatuses known in the art for the coating of low viscosities hot melt compositions in a layer having a required thickness onto a substrate, while also keeping the advantageous characteristics of the preferred thermoplastic hydrophilic polymers in providing hydrophilic continuous moisture vapour permeable, liquid impermeable layers or films.

Thermoplastic hydrophilic polymeric compositions having such viscosities can also provide very thin films or layers.

Suitable compatible plasticisers comprised in the thermoplastic hydrophilic polymeric composition according to this preferred embodiment of the present invention include citric acid esters, tartaric acid esters, glycerol and its esters, sucrose esters, adipates, sebacates, sorbitol, epoxidized vegetal oils, polymerised vegetal oils, polyols, phthalates, liquid polyesters, glycolates, p-toluene sulfonamide and derivatives, glycols and polyglycols and their derivatives, sorbitan esters, phosphates, monocarboxylic fatty acids (C₈-C₂₂) and their derivatives, and mixtures thereof.

According to a particularly preferred embodiment of the present invention particularly preferred plasticisers are hydrophilic plasticisers such as acids, esters, amides, alcohols, polyalcohols, or mixtures thereof as disclosed in our application WO 99/64505. Said particularly preferred hydrophilic plasticisers provide the further advantage that they do not impair, and possibly can even enhance, the moisture vapour permeability of a resulting layer or film formed from the preferred thermoplastic hydrophilic polymeric composition of the present invention comprising said plasticiser or blend of plasticisers, when compared to a corresponding film or layer formed from a thermoplastic hydrophilic polymeric composition comprising the same components, but without the plasticiser or plasticisers.

These particularly preferred hydrophilic plasticiser or blend of hydrophilic plasticisers can of course also adjust the viscosity of the thermoplastic composition according to a preferred embodiment of the present invention to the preferred values in order to make it processable by coating said thermoplastic composition onto a substrate in a layer or film having a desired thickness.

Suitable preferred hydrophilic plasticisers according to this preferred embodiment of the present invention comprise acids, esters, amides, alcohols, polyalcohols, or mixtures thereof, wherein particularly preferred hydrophilic plasticisers are citric acid esters, tartaric acid esters, glycerol and its esters, sorbitol, glycolates, and mixtures thereof.

It is possible that some of the plasticisers comprised in the above list, and disclosed in the above mentioned patent applications WO 99/64077 and WO 99/64505, actually do not satisfy the condition in terms of solubility in water at 25°C according to the present invention, but this is readily verifiable by the man skilled in the art when selecting a suitable plasticiser or blend of plasticisers according to the present invention, based on e.g. data available in literature, or simple experimentation.

The thermoplastic hydrophilic polymeric compositions of the present invention may in addition comprise additional optional components to further improve the processability of the compositions and also the mechanical characteristics as well as other characteristics such as tackiness, resistance to ageing by light and oxygen, visual appearance etc., of the films or layers formed from such thermoplastic polymeric hydrophilic compositions.

Such optional components include tackifying resins or blends of tackifying resins typically having a softening point of 125°C or less. Preferred resins may be selected from rosins and rosin esters, hydrocarbon resins, aliphatic resins, terpene and terpene-phenolic resins, aromatic resins, synthetic C₅ resins, mixtures of synthetic C₅-C₉ resins, and mixtures thereof. Other optional components of said thermoplastic compositions include anti-oxidants, anti-ultraviolets, pigments and mixtures thereof, which may be present within the composition at a level of up to 10% by weight of the composition.

Preferably the thermoplastic polymeric hydrophilic composition of the present invention comprises from 5% to 99%, preferably from 20% to 70%, more preferably from 30% to 50%, by weight of the thermoplastic polymeric hydrophilic composition, of the thermoplastic hydrophilic polymer or mixture of thermoplastic hydrophilic polymers, and from 1% to 95%, preferably from 10% to 70%, more preferably from 20% to 50%, by weight of the thermoplastic hydrophilic composition, of the suitable compatible plasticiser or blend of plasticisers.

More preferably the thermoplastic polymeric hydrophilic composition of the present invention also comprises from 0% to 60%, preferably from 10% to 50%, more preferably from 20% to 40% by weight of the thermoplastic polymeric hydrophilic composition, of a compatible tackifying resin, or blend of tackifying resins.

A thermoplastic hydrophilic polymeric composition according to the present invention can be manufactured with any known process that will typically comprise the steps of providing at least the thermoplastic hydrophilic polymer or mixture of polymers and the suitable compatible plasticiser or blend of plasticisers, and optionally any further additional components as explained above, such as for example a compatible tackifying resin or a blend of tackifying resins, heating the components and compounding them, e.g. with a known suitable mixer to form the thermoplastic hydrophilic polymeric composition in the molten state for subsequent process steps.

Alternatively, solvent or emulsion systems can be created and used to process the thermoplastic hydrophilic polymeric compositions of the present invention, either as intermediate or final step in making moisture vapour permeable, liquid impermeable structures from said compositions, and articles comprising said structures.

According to the present invention a moisture vapour permeable, liquid impervious layer can be formed from the thermoplastic hydrophilic polymeric composition of the present invention, for example by laying said thermoplastic hydrophilic polymeric composition onto a substrate. The films or layers formed from the thermoplastic compositions of the present invention preferably have a moisture vapour transmission rate of at least 100 g/m²·24h, preferably at least 300 g/m²·24h, more preferably at least 400 g/m²·24h, most preferably at least 500 g/m²·24h, with a thickness of said layer or film of at least 0.5 µ, preferably of at least 15 µ, more preferably of at least 25 µ, said preferred thicknesses depending on the selected end use for said films or layers, said water vapour transmission rate measured according to the modified ASTM E-96 "Upright Cup" Method.

According to the present invention, films or layers can be formed from the thermoplastic hydrophilic polymeric compositions described so far which have a thickness of from about 0.5 µ to about 200 µ and above, said films or layers being usable as such, or in combination with different substrates, such as for example in a layered structure comprising a nonwoven fibrous substrate.

More in general, the thickness of the structures formed from the thermoplastic hydrophilic polymeric compositions of the present invention can be constant or vary within the structure. Though not limited to any specific thickness range, depending upon application there may be preferred ranges. For example, the preferred range for a structure comprised in a disposable article may desirously range from as thick as 400 microns down to less than 0.5 microns and more preferably, in certain cases, substantially less than 0.5 microns. In contrast, a construction or even packaging application may, for certain reasons, dictate a preferred range from 200 to 2000 microns or even thicker for the structure.

A process for making a layer or film from a thermoplastic polymeric hydrophilic composition according to the present invention typically comprises the steps of providing said composition, heating it to make it flowable, and forming said composition in the molten, semi-molten, or plastic state onto a substrate in a layer or film having the desired thickness, e.g. with a film extrusion process, or with a hot melt coating process, depending on the viscosity achieved for the composition at the process conditions, as explained above. While in principle said substrate can be simply a formation substrate, onto which the thermoplastic hydrophilic polymeric composition is formed in order to make a film or layer of the desired thickness which is subsequently separated from said substrate and used as such, in a preferred embodiment of the present invention a moisture vapour permeable, water impervious composite structure can be formed which comprises the thermoplastic hydrophilic polymeric composition and a suitable substrate onto which said thermoplastic composition is laid, wherein the substrate is also preferably moisture vapour permeable.

Other known processes can be used for making moisture vapour permeable, liquid impermeable structures, not limited to films and layers, from the thermoplastic hydrophilic polymeric compositions of the present invention, and articles comprising said structures.

A class of such methods is generally described as "moulding" where the material is often shaped via use of male or female moulds or combinations of moulds. Depending on the technique, certain processing temperature and pressure (or vacuum) conditions may be preferred for production of a given structure or article. Such known moulding methods include, but are not limited to: dip moulding, blow moulding, injection moulding, compression moulding, thermoforming, vacuum thermoforming, extrusion moulding, rotational moulding, slush moulding, etc.

Other known methods for processing the thermoplastic hydrophilic polymeric compositions of the present invention also include: film and sheet casting; blown film techniques; an additional tentering process step; an additional calendering step; an additional quenching step; an additional heat treatment step; etc. The nature of the specific production conditions or type or order of process steps will vary depending on the chosen making technique, environmental condition, material format, etc. For example, a process step may need to be included to remove: (i) solvent if a solvent-based format of the raw material form of the thermoplastic hydrophilic polymeric composition is chosen; (ii) water if an emulsion-based format of the raw material form of the thermoplastic hydrophilic polymeric composition is chosen; or, (iii) heat if a hot melt format of the raw material form of the thermoplastic hydrophilic polymeric composition is chosen.

A film or sheet can be produced with two or more layers where at least one of the layers comprises the thermoplastic hydrophilic polymeric composition of this invention. This can be accomplished by a variety of known means, including but not limited to: co-extrusion, extrusion coating, etc.

While it may be at times preferable that the entire structure or article be comprised solely of the thermoplastic hydrophilic polymeric composition of the present invention, the structure or the article can be a composite with one or more other materials. The composite, for example, can involve two or more components of the specific thermoplastic hydrophilic polymeric composition of the present invention or different specific thermoplastic hydrophilic polymeric compositions of the present invention.

Alternatively, the composite can involve at least one component of the thermoplastic hydrophilic polymeric composition in combination with one or more other materials. Such materials include, but are not limited to: fibres, fibrous batts, non-wovens, wovens, papers, metal foils, micro-porous or porous membranes, films such as polymeric films, inorganic structures such as compressed gypsum sheets, perforated or apertured films and papers, macroscopically expanded films, cloth, substantially rigid fibre-based materials such as lumber, etc.

Said other components may be non-absorbent, absorbent, liquid-containing, etc.

The thermoplastic hydrophilic polymeric compositions of the present invention can also be manufactured as a foam, including closed cell foams, with known means, for example to form cellular foam structures.

Another useful technique is the process of spray coating. The thermoplastic hydrophilic polymeric composition of this invention lends itself to a heated spraying technique whereas upon heating the viscosity is sufficiently lowered to allow spray coating or sputtering. Such thermoplastic hydrophilic polymeric composition spray coating can occur with the aid of a mould, either male or female, to build surfaces or walls of the article. Afterward, the article and mould (or mould parts) are separated from each other. Alternately, the spray coating method can employ different starting raw material formats of the polymer composition such as a solvent-based approach or an emulsion.

For a composite article comprising the thermoplastic hydrophilic polymeric composition of the present invention, and employing the spray coating approach, the other material may provide sufficient structure by itself such that the other material acts as the mould, after which it is sufficiently coated the composite article is complete, avoiding the before-mentioned separation of article from mould.

The selected high water solubility of the suitable compatible plasticiser or blend of suitable compatible plasticisers in the thermoplastic hydrophilic polymeric compositions of the present invention allows a controlled extraction/release of said plasticiser or plasticiser by means of direct contact of said thermoplastic hydrophilic polymeric compositions with water or aqueous fluids, which can be achieved right after formation of the thermoplastic hydrophilic polymeric composition into a structure, e.g. into a film or layer, for example as a further manufacture step, or alternatively during the use of a structure, or of an article comprising said structure, made of the thermoplastic hydrophilic polymeric composition according to the present invention.

According to an embodiment of the present invention, a thermoplastic hydrophilic polymeric composition can be compounded which comprises a percentage of plasticiser or plasticisers higher than that actually desired for the final composition itself, e.g. in order to further facilitate processing by suitably reducing the viscosity of the thermoplastic polymeric hydrophilic composition at the process conditions, as explained above. After formation of the thermoplastic hydrophilic polymeric composition into a structure, e.g. into a film or layer, the excess amount of plasticiser or plasticisers can be extracted from the structure by means of direct contact with water, e.g. in a water bath, in order to modify the mechanical characteristics of the composition, and in turn of the structure made thereof. For example a controlled extraction of a plasticiser or plasticisers is typically effective in order to increase the stiffness, the resistance to temperature, the elastic modulus of the composition, and of the structure made thereof. The level of the extraction can be controlled, for example by varying the duration and/or the temperature of the water bath, in order to achieve the desired change in the mechanical characteristics, depending on the final use intended for the structure made of the thermoplastic hydrophilic polymeric composition.

Alternatively, a controlled extraction/release of a plasticiser or plasticisers from a thermoplastic hydrophilic polymeric composition according to the present invention, e.g. formed into a structure such as a water vapour permeable, liquid impermeable layer or film, can also occur during the use of the structure, or of an article comprising the structure, by direct contact of the thermoplastic hydrophilic polymeric composition with water or aqueous fluids. This can be advantageous in case the plasticiser or plasticisers have some activity towards surfaces or objects intended to come in contact with the structure. Extraction by water or aqueous fluids during use can bring said water or aqueous fluids carrying in solution the plasticiser or plasticisers in contact with said surface or object, in order to perform this activity of the plasticiser or plasticisers.

In an embodiment of the present invention a moisture vapour permeable, liquid impervious composite layered structure can be provided wherein the contribution of the layer formed from the thermoplastic hydrophilic polymeric composition of the present invention to the overall performance of the composite material can only reside in the provision of a breathable liquid barrier and hence could be advantageously provided as thinly as possible. The remaining performance physical criterion is then preferably provided by the provided substrate, that therefore preferably acts also as a support layer.

The substrate, or support layer may be any useful layer which is preferably also moisture vapour permeable, preferably having a moisture vapour permeability of at least 100 g/m²·24h, more preferably at least 300 g/m²·24h, and most preferably at least 500 g/m²·24h.

Suitable substrates for use herein as support layers include two dimensional, planar micro and macro-porous films; macroscopically expanded films; formed apertured films; nonwoven and woven layers. According to the present invention the apertures in said layer may be of any configuration, but are preferably spherical or oblong and may also be of varying dimensions. The apertures preferably are evenly distributed across the entire surface of the layer, however layers having only certain regions of the surface having apertures are also envisioned.

Suitable two dimensional porous planar layers of the backsheet may be made of any material known in the art, but are preferably manufactured from commonly available polymeric materials. Suitable materials are for example Goretex™ or Sympatex™ type materials well known in the art for their application in so-called breathable clothing. Other suitable materials include XMP-1001 of Minnesota Mining and Manufacturing Company, St. Paul, Minnesota, USA and Exxaire XBF-101W, supplied by the Exxon Chemical Company. As used herein the term two dimensional planar layer refers to layers having a depth of less than 1 mm, preferably less than 0.5 mm, wherein the apertures have an average uniform diameter along their length and which do not protrude out of the plane of the layer. The apertured materials for use as a backsheet in the present invention may be produced using any of the methods known in the art such as described in EPO 293 482 and the references therein. In addition the dimensions of the apertures produced by this method may be increased by applying a force across the plane of the backsheet layer (i.e. stretching the layer).

Suitable apertured formed films include films which have discrete apertures which extend beyond the horizontal plane of the garment facing surface of the layer towards the core thereby forming protuberances. The protuberances have an orifice located at its terminating end. Preferably said protuberances are of a funnel shape, similar to those described in US 3,929,135. The apertures located within the plane and the orifices located at the terminating end of protuberance themselves maybe circular or non circular provided the cross sectional dimension or area of the orifice at the termination of the protuberance is smaller than the cross sectional dimension or area of the aperture located within the garment facing surface of the layer. Preferably said apertured performed films are uni directional such that they have at least substantially, if not complete one directional fluid transport towards the core.

Suitable macroscopically expanded films for use herein include films as described in for example in US 4,637,819 and US 4,591,523.

Preferred support layers for use herein include woven and nonwoven layers, most preferably hydrophobic fibrous layers such as hydrophobic nonwovens.

The composite layered structures of this preferred embodiment of the present invention are particularly advantageous as they allow the possibility of providing a composite wherein the thermoplastic composition may be formed onto the support substrate as a layer with the desired thickness. By e.g. suitably tailoring the viscosity of the thermoplastic hydrophilic polymeric composition at the process conditions as explained above, typical coating conditions and apparatuses known in the art for the direct coating of low viscosities hot melts can be readily utilised in order to provide the thermoplastic hydrophilic polymeric composition at the desired thickness onto the substrate. Alternatively, other known processes such as film extrusion can be used in case of thermoplastic hydrophilic polymeric compositions according to the present invention having a higher viscosity at the process conditions.

A possible method for forming a composite laminate by coating the thermoplastic composition onto a substrate acting as a support layer is described in PCT application WO 96/25902.

At least at the coating temperature, the thermoplastic composition in form of a layer preferably exhibits adhesive properties on the supportive substrate in order to form the preferred composite such that no additional adhesive is required to achieve a permanent attachment between the thermoplastic composition and the substrate. In some applications it may be also desirable that the thermoplastic composition remains tacky at any temperature i.e. it is formulated so to have the typical characteristics of a pressure sensitive adhesive.

The thermoplastic hydrophilic polymeric compositions of the present invention and the moisture vapour permeable, liquid impervious structures, e.g. layers and composites, formed therefrom find utility in a number of applications wherein liquid imperviousness and moisture vapour permeability are desirable. In particular the present invention can be effectively utilised within absorbent articles such as diapers, sanitary napkins, panty liners, incontinence products and breast pads; perspiration pads such as underarm-, wrist- and head perspiration pads, collar inserts, shoe inserts, hat bands; protecting bedding covers, protective clothing, wrapping material for e.g. food or other goods, and the like.

Other articles comprising the thermoplastic hydrophilic polymeric compositions of the present invention, also comprise hand coverings such as gloves, finger cots, mitts, mittens; foot or leg coverings such as socks, hose, pantyhose, shoes, slippers; head coverings such as hats, caps; prophylactic articles such as condoms, semen shields internally placed inside the vaginal cavity; face coverings such as face masks, nose covers, ear covers or mitts; body support items such as male organ "athletic" supporters, brassieres; formed clothing for use as underwear, protective sleeves, or as a part of or wholly incorporated into protective pads. Other example articles and applications include but are not limited to: flexible or drapable clothing articles for humans or other living creatures such as the non-limiting examples of shirts, pants, undergarments, bibs, smocks, coats, scarves, body wraps, stockings, leggings, skirts, dresses, etc.; other flexible or drapable clothing for various tasks and occupations including medical professions, agricultural jobs, mechanical assembly and repair, emergency public services, the military, athletic endeavours, cleaning positions, etc.

Another example category of use involves packaging such as with food products such as fresh produce and baked goods (bread, rolls, cakes) as non-limiting examples.

A further example category of use involves agriculture and horticulture such as, as non-limiting examples, an individual article (container, three dimensional "bag") which is placed to partially or totally enclose an individual or specific group of plants.

An even further example category of use involves protective furniture coverings such as protective covers for upholstered chairs and sofas, etc.

Articles comprising the thermoplastic hydrophilic polymeric compositions of the present invention can be generally flexible or rigid.

All such articles can also be typically disposable. Preferably the moisture vapour permeable, liquid impervious structures, e.g. layers and composites, formed from the thermoplastic compositions of the present invention have an overall moisture vapour transfer rate of at least 100 g/m²·24h, preferably of at least 300 g/m²·24h, and more preferably at least 500 g/m²·24h.

A moisture vapour permeable, liquid impervious composite structure formed by forming the thermoplastic polymeric hydrophilic composition of the present invention onto a suitable substrate finds particular utility as the backsheet for disposable absorbent articles, especially sanitary napkins and panty liners, but also diapers, incontinence products and breast pads. Such articles will typically comprise components known to the skilled person such as a liquid pervious topsheet, an absorbent core and a backsheet and may optionally also comprise other components such as fastening means, wings, and the like.

According to the present invention the complex viscosity η∗ is measured using a Rheometer RDA-II available from Rheometrics Co. Moisture vapour permeability is measured as Water Vapour Transmission Rate (WVTR) at 23°C and 50% relative humidity according to the modified ASTM E-96 "Upright Cup" method. The only modification to the standard ASTM E-96 "Upright Cup" method consists in a change in the height of the air gap between the sample and the water surface in the cup, which height is 3 mm ± 0.5 mm, instead of 19 mm ± 2.5 mm, as specified in the standard test method.

## Claims

1. A thermoplastic hydrophilic polymeric composition comprising:
a thermoplastic hydrophilic polymer or a mixture of thermoplastic hydrophilic polymers, said thermoplastic hydrophilic polymer, or alternatively each of said thermoplastic hydrophilic polymers of said mixture selected from the group consisting of polyurethanes, poly-ether-amides block copolymers, polyethylene-acrylic acid copolymers, polyethylene oxide and its copolymers, ethylene acrylic esters copolymers, poly lactide and copolymers, polyamides, polyester block copolymers, sulfonated polyesters, poly-ether-ester block copolymers, poly-ether-ester-amide block copolymers, polyacrylates, polyacrylic acids and derivatives, ionomers, polyethylene-vinyl acetate with a vinyl acetate content of more than 28% by weight, polyvinyl alcohol and its copolymers, polyvinyl ethers and their copolymers, poly-2-ethyl-oxazoline and derivatives, polyvinyl pyrrolidone and its copolymers, thermoplastic cellulose derivatives, or mixtures thereof;
a suitable compatible plasticiser or a blend of suitable compatible plasticisers,
said thermoplastic hydrophilic polymeric composition **characterized in that**:
said suitable compatible plasticiser has a solubility in water at 25°C of more than 6 g/100 ml H₂O, preferably of more than 7 g/100 ml H₂O, more preferably of more than 9 g/100 ml H₂O
or alternatively, said blend of suitable compatible plasticisers contains more than 50% by weight of said blend of at least a plasticiser having a solubility in water at 25°C of more than 6 g/100 ml H₂O, preferably of more than 7 g/100 ml H₂O, more preferably of more than 9 g/100 ml H₂O.

2. A thermoplastic hydrophilic polymeric composition according to claim 1, wherein said thermoplastic hydrophilic polymer or said mixture of thermoplastic hydrophilic polymers comprises thermoplastic poly-ether-amide block copolymers, thermoplastic poly-ether-ester-amide block copolymers, thermoplastic polyester block copolymers, thermoplastic polyurethanes, or mixtures thereof.

3. A thermoplastic hydrophilic polymeric composition according to any preceding claim, wherein said suitable compatible plasticiser, or alternatively each plasticiser of said blend of suitable compatible plasticisers is selected from the group consisting of citric acid esters, tartaric acid esters, glycerol and its esters, sucrose esters, adipates, sebacates, sorbitol, epoxidized vegetal oils, polymerised vegetal oils, polyols, phthalates, liquid polyesters, glycolates, p-toluene sulfonamide and derivatives, glycols and polyglycols and their derivatives, sorbitan esters, phosphates, monocarboxylic fatty acids (C₈-C₂₂) and their derivatives, and mixtures thereof.

4. A thermoplastic hydrophilic polymeric composition according to claim 3, wherein said suitable compatible plasticiser, or alternatively each plasticiser of said blend of suitable compatible plasticisers is selected from the group consisting of citric acid esters, tartaric acid esters, glycerol and its esters, sorbitol, glycolates, and mixtures thereof.

5. A thermoplastic hydrophilic polymeric composition according to any preceding claim, wherein said thermoplastic hydrophilic polymeric composition comprises:
from 5% to 99%, preferably from 20% to 70%, more preferably from 30% to 50%, by weight of said thermoplastic hydrophilic composition, of said thermoplastic hydrophilic polymer or mixture of thermoplastic hydrophilic polymers,
from 1% to 95%, preferably from 10% to 70%, more preferably from 20% to 50%, by weight of said thermoplastic polymeric hydrophilic composition, of said suitable compatible plasticiser, or blend of suitable compatible plasticisers, and
from 0% to 60%, preferably from 10% to 50%, more preferably from 20% to 40%, by weight of said thermoplastic polymeric hydrophilic composition, of a compatible tackifying resin, or blend of compatible tackifying resins.

6. A thermoplastic hydrophilic polymeric composition according to claim 5, wherein said compatible tackifying resin, or alternatively each tackifying resin of said blend of tackifying resins is selected from the group consisting of rosins and rosin esters, hydrocarbon resins, aliphatic resins, terpene and terpene-phenolic resins, aromatic resins, synthetic C₅ resins, mixtures of synthetic C₅-C₉ resins, and mixtures thereof.

7. A moisture vapour permeable layer formed from the thermoplastic hydrophilic polymeric composition of any of claims 1 to 6, wherein said layer is liquid impervious and has a water vapour transmission rate of (WVTR) of at least 100 g/m²·24h, preferably of at least 300 g/m²·24h, more preferably of at least 400 g/m²·24h, most preferably of at least 500 g/m²·24h with a thickness of said layer of at least 15 µm, said water vapour transmission rate measured according to the modified ASTM E-96 "Upright Cup" method.

8. A moisture vapour permeable, liquid impervious layered structure comprising the layer of claim 7 bonded to a substrate, said substrate being moisture vapour permeable.

9. An absorbent article such as a diaper, a sanitary napkin, a panty liner, an incontinence product, a breast pad, said absorbent article comprising a moisture vapour permeable, liquid impervious layer or layered structure according to claims 7 or 8.
